# EUROPEAN PATENT APPLICATION

(11) **EP 1 510 215 A1**
(43) Date of publication of application: **02.03.2005**
(21) Application number: 03730781.6
(22) Date of filing: 02.06.2003
(51) Int. Cl.: A61K 31/704, A61K 9/14, A61K 47/04, A61K 47/12, A61K 47/26, A61K 47/34, A61K 9/08, A61K 9/107, A61P 35/00

(54) **NOVEL SOLID PREPARATION CONTAINING BLOCK COPOLYMER AND ANTHRACYCLINE ANTICANCER AGENT AND PROCESS FOR PRODUCING THE SAME**

(30) Priority: 03.06.2002 JP 2002161263
(71) Applicant: Nippon Kayaku Kabushiki Kaisha, Tokyo 102-8172 (JP); Japan Science and Technology Agency, Kawaguchi-shi, Saitama 332-0012 (JP); Sakurai, Yasuhisa, Suginamu-ku, Tokyo 168-0064 (JP)
(72) Inventor: MOTOYAMA, Jun, Kita-ku, Tokyo 115-0042 (JP)
(74) Representative: Wablat, Wolfgang, Dr.Dr.
(86) International application number: PCT/JP2003/006945
(87) International publication number: WO 2003/101465

(57) **Abstract**

There has been required a clinically applicable solid preparation for injection which contains a block copolymer composed of a hydrophilic polymer structure moiety and a hydrophobic polyamino acid structure moiety and an anthracycline anticancer agent. It is intended to provide a clinically applicable solid preparation for injection which contains a block copolymer composed of a hydrophilic polymer structure moiety and a hydrophobic polyamino acid structure moiety, an anthracycline anticancer agent, a saccharide and a base.

## Description

### FIELD OF THE INVENTION

The present invention relates to a solid preparation for injection comprising a block copolymer composed a hydrophilic polymer structure moiety and a hydrophobic polyamino acid structure moiety, an anthracycline anticancer agent as a therapeutic agent for malignant tumors, and a method of producing the same.

### BACKGROUND ART

There are known some preparations obtained by allowing an anthracycline anticancer agent to be contained in a polymer micelle formed by a block copolymer composed of a hydrophilic polymer structure moiety having polyethylene oxide as a main chain and a polyamino acid structure moiety having a fat-soluble substituent at a side chain.

For example, JP-A No. 7-69900 describes a micelle preparation aqueous solution comprising a block copolymer composed of polyethylene oxides and polyaspartic acid bonded by doxorubicin residue, and doxorubicin.

However, this publication has no specific description regarding a clinically applicable solid preparation for injection. Problems of a method of producing the above-mentioned aqueous solution include processes of irradiation with ultrasonic wave and dialysis which are not easily carried out industrially, and uses of organic solvents such as dimethylformamide in dissolving a block copolymer and doxorubicin, and isopropyl ether and the like in precipitating a block copolymer. When an organic solvent is used, complete removal of the solvent, and an apparatus having corrosion resistance against the solvent used and an explosion preventing property, are necessary, and safety of operators against a vapor of the solvent should be considered, therefore, an equipment for producing a preparation for injection would be a very special one. Consequently, an organic solvent is not used frequently in a manufacturing plant of preparations for injection.

Therefore, there is desired a clinically applicable solid preparation for injection comprising a block copolymer composed of a hydrophilic polymer structure moiety and a hydrophobic polyamino acid structure moiety, and an anthracycline anticancer agent, useful as a therapeutic agent for malignant tumors, showing stability for a long period of time, providing safety of additives, and which can be produced by a usual production apparatus requiring no dialysis process and using no organic solvent.

### DISCLOSURE OF THE INVENTION

The inventor of the present invention has been dedicated to studying to solve the above-mentioned problems, and resultingly found a novel solid preparation for injection and a method of producing the same, completing the present invention. Namely, the present invention relates to
(1) A solid preparation for injection comprising a block copolymer composed a hydrophilic polymer structure moiety and a hydrophobic polyamino acid structure moiety, an anthracycline anticancer agent, a saccharide and a base.
(2) The solid preparation for injection according to (1), wherein the anthracycline anticancer agent is doxorubicin or its salt and the saccharide is disaccharide.
(3) The solid preparation for injection according to (1) or (2), wherein the saccharide includes one or more compounds selected from the group consisting of sucrose, trehalose, maltose and lactose.
(4) The solid preparation for injection according to any one of (1) to (3), wherein the base includes one or more compounds selected from the group consisting of sodium hydrogen carbonate, disodium hydrogen phosphate, sodium citrate and sodium hydroxide.
(5) The solid preparation for injection according to any of (1) to (4), wherein the hydrophilic polymer structure moiety in the block copolymer is a polyethylene oxide derivative, and the hydrophobic polyamino acid structure moiety is polyaspartic acid having a side chain to which an anthracycline anticancer agent is bonded.
(6) The solid preparation for injection according to (5), wherein the anthracycline anticancer agent bonded to a side chain of the hydrophobic polyamino acid structure moiety is doxorubicin.
(7) The solid preparation for injection according to (1) , wherein the content of the anthracycline anticancer agent is 10 to 100 parts by weight, the content of the saccharide is 10 to 500 parts by weight and the content of the base is 0.1 to 10 parts by weight, based on 100 parts by weight of the block copolymer composed a hydrophilic polymer structure moiety and a hydrophobic polyamino acid structure moiety.
(8) The solid preparation for injection according to (1), wherein the proportions of the components are as described below:

| | |
|---|---|
| block copolymer composed a hydrophilic polymer structure moiety and a hydrophobic polyamino acid structure moiety | 10 to 85 parts by weight |
| anthracycline anticancer agent | 1 to 50 parts by weight |
| saccharide | 5 to 80 parts by weight |
| base | 0.05 to 5 parts by weight. |

(9) The solid preparation for injection according to (8), wherein the anthracycline anticancer agent is doxorubicin or salt thereof, the saccharide is sucrose, trehalose, maltose or lactose, and the base is sodium hydrogen carbonate, disodium hydrogen phosphate, sodium citrate or sodium hydroxide.
(10) The solid preparation for injection according to any one of (1) to (9), wherein the solid preparation for injection is a lyophilized preparation.
(11) A block copolymer micelle preparation composed of a hydrophilic polymer structure moiety and a hydrophobic polyamino acid structure moiety containing an anthracycline anticancer agent in a hydrophobic inner core, obtained by dissolving the solid preparation for injection according to any one of (1) to (10) in infusion.
(12) A block copolymer micelle solution of pH 4 to 9, comprising a block copolymer composed of a hydrophilic polymer structure moiety and a hydrophobic polyamino acid structure moiety bonded by an anthracycline anticancer agent, an anthracycline anticancer agent, a saccharide, and a base.
(13) A process for producing a solid preparation for injection, comprising dissolving a block copolymer composed of a hydrophilic polymer structure moiety and a hydrophobic polyamino acid structure moiety, an anthracycline anticancer agent, a saccharide and a base in water, and then drying them to give solid.
(14) The process for producing a solid preparation for injection according to (13), wherein in dissolving a block copolymer composed of a hydrophilic polymer structure moiety and a hydrophobic polyamino acid structure moiety in water, a base is added and the mixture is heated.
(15) The process for producing a solid preparation for injection according to (14), wherein the heating temperature is 50 to 80°C.
(16) The process for producing a solid preparation for injection according to any one of (13) to (15), comprising dissolving a block copolymer composed of a hydrophilic polymer structure moiety and a hydrophobic polyamino acid structure moiety, an anthracycline anticancer agent, a saccharide and a base in water, and then filtrating through a membrane filter.
(17) The process for producing a solid preparation for injection according to (16), wherein the membrane filter is a cellulose-based or synthetic polymer-based filter and its pore size is 0.05 to 0.5 µm.
(18) The micelle solution according to (12), wherein the anthracycline anticancer agent is doxorubicin or its salt and the saccharide is disaccharide.
(19) The micelle solution according to (18), wherein the disaccharide includes one or more compounds selected from the group consisting of sucrose, trehalose, maltose and lactose.
(20) The micelle solution according to (12), wherein the base includes one or more compounds selected from the group consisting of sodium hydrogen carbonate, disodium hydrogen phosphate, sodium citrate and sodium hydroxide.
(21) The micelle solution according to (12), wherein the anthracycline anticancer agent is doxorubicin or its salt, the saccharide is sucrose, trehalose, maltose or lactose, and the base is sodium hydrogen carbonate, disodium hydrogen phosphate, sodium citrate or sodium hydroxide.
(22) The micelle solution according to (12), wherein the block copolymer is composed of a hydrophilic polymer structure moiety and a hydrophobic polyamino acid structure moiety bonded by an anthracycline anticancer agent.
(23) The micelle solution according to (22), wherein the hydrophilic polymer structure moiety of the block copolymer is a polyethylene oxide derivative and the hydrophobic polyamino acid structure moiety is polyaspartic acid having a side chain to which an anthracycline anticancer agent is bonded.
(24) The micelle solution according to (22) or (23), wherein the anthracycline anticancer agent bonded to a side chain of the hydrophobic polyamino acid structure moiety is doxorubicin.
(25) A solid preparation for injection obtained by drying the micelle solution according to any one of (18) to (24).

### BEST MODES FOR CARRYING OUT THE INVENTION

The solid preparation for injection of the present invention comprises a block copolymer composed a hydrophilic polymer structure moiety and a hydrophobic polyamino acid structure moiety, an anthracycline anticancer agent, a saccharide and a base.

The anthracycline anticancer agent used in the solid preparation for injection of the present invention is not particularly restricted, providing it is an anthracycline compound having an anticancer activity, and specifically, anthracycline antibiotics and their derivatives used in clinical treatment of cancers are mentioned, and examples thereof include daunorubicin, doxorubicin, pyrarubicin, aclarubicin, epirubicin, idarubicinand the like. Particularly preferable is doxorubicin.

When the anthracycline anticancer agent used in the present invention is an anthracycline anticancer agent capable of forming a salt with an acid or base, use of such a salt is also included in the scope of the present invention. As the salt, an acid-added salt is preferable, and as the acid for forming an acid-addition salt, for example, hydrochloric acid, sulfuric acid, nitric acid, hydrobromic acid or the like.

As the polymer constituting a hydrophilic polymer structure moiety in the present invention, those showing hydrophilicity usually known are all included, however, specific examples thereofincludepolyethylene oxide, polysaccharide,polyacrylic acid, polyvinylpyrrolidone, polyvinyl alcohol and derivatives thereof and the like, and particularly preferable are polyethylene oxide derivatives.

The hydrophobic polyamino acid structure moiety in the present invention is not particularly restricted, providing it is an α-amino acid or β-amino acid or derivative thereof showing hydrophobicity, and preferable are polyamino acids in which an anthracycline anticancer agent is bonded to a part or all of side chains, and particularly, preferable is polyaspartic acid in which doxorubicin is bonded to a part of side chains.

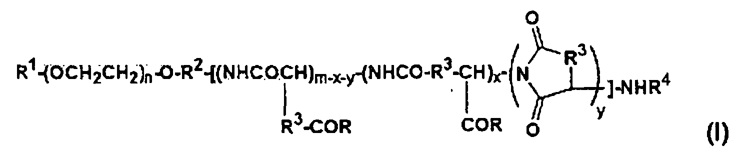

The block copolymer composed of a hydrophilic polymer structure moiety and a hydrophobic polyamino acid structure moiety used in the present invention is produced, for example, by the following method. The block copolymer produced by this method is guessed to be represented, for example, by the above-mentioned general formula (I). In the formula, R represents hydroxyl group or anthracycline anticancer agent residue, R¹ represents hydrogen atom or lower alkyl group, preferably lower alkyl group, R² represents lower alkylene group, R³ represents methylene group or ethylene group, and R⁴ represents hydrogen atom or lower acyl group, preferably lower acyl group. n represents an integer of 5 to 1000, m represents an integer of 2 to 300, x+y represents an integer of 0 to 300, preferably, n is 80 to 300, m is 20 to 50, and x+y is 0 to 50, and x+y is not larger than m. x and y can be any value including 0, providing it is an integer satisfying the above-mentioned condition.

The anthracycline anticancer agent in the anthracycline anticancer agent residue represented by R is the same as the anthracycline anticancer agent mentioned as the anthracycline anticancer agent used in the solid preparation for injection of the present invention described above, and specific examples of the anthracycline anticancer agent are also the same as described above, and the preferable anthracycline anticancer agent is doxorubicin as in the above case. The weight proportion of an anthracycline anticancer agent residue in a hydrophobic polyamino acid structure moiety in one molecule of a block copolymer is preferably 20% to 70%, particularly preferably 25% to 60%.

The lower alkyl group in R¹ includes straight chain or branched alkyl groups having 1 to 4 carbon atoms, and specific examples thereof include methyl group, ethyl group, n-propyl group, i-propyl group, n-butyl group, t-butyl group and the like, and preferable is methyl group.

The lower alkylene group in R² includes straight chain or branched alkylene groups having 1 to 4 carbon atoms, and specific examples thereof include methylene group, ethylene group, trimethylene group, 2-methyltrimethylene group, tetramethylene group and the like, and preferable is trimethylene group.

R³ represents methylene group or ethylene group, and preferable is methylene group.

The lower acyl group in R⁴ includes acyl group having 1 to 4 carbon atoms, and specific examples thereof include formyl group, acetyl group, propionyl group, butyryl group and the like, and preferable is acetyl group.

The bonding mode of an anthracycline anticancer agent residue with a side chain of a polyamino acid in the general formula (I) is not particularly restricted, and preferable is an amide bond or ester bond formed by an amino group or hydroxyl group of an anthracycline anticancer agent and a carboxylic acid side chain of a polyamino acid, and particularly preferable is an amide bond formed by a primary amino group at an amino sugar portion of an anthracycline anticancer agent and a carboxylic acid side chain of a polyamino acid.

As the block copolymer composed of a hydrophilic polymer structure moiety and a hydrophobic polyamino acid structure moiety in the present invention, block copolymer is particularly preferably mentioned in which R¹ represents a methyl group, R² represents a trimethylene group, R³ represents a methylene group, R⁴ represents an acetyl group, n is 80 to 300, m is 20 to 50 and x+y is 0 to 50 not larger than m.

The block copolymer composed of a hydrophilic polymer structure moiety and a hydrophobic polyamino acid structure moiety used in the present invention may be that produced according to a method described, for example, in JP-A No. 7-69900. Namely, one-end methoxy one-end aminopropoxy polyethylene glycol and β-benzyl-L-aspartate-N-carboxylic anhydride are reacted, a terminal amino group of the resulting block copolymer is acylated, a side chain benzyl ester is hydrolyzed with an alkali, and the resulting free carboxylic acid at a side chain and an anthracycline anticancer agent are condensed using a condensin agent and a reaction aid, to obtain the block copolymer.

The saccharide used in the solid preparation for injection of the present invention is not particularly restricted, providing it is a saccharide capable of being used as a stabilizing agent, dissolution auxiliary agent or excipient, but a disaccharide is preferable, and specific examples thereof include sucrose, maltose, lactose, trehalose and the like, and sucrose and trehalose are more preferable. Two or more of these disaccharides may be combined for use. Further, one or more sugar alcohols may be combined with them. As the sugar alcohol, inositol, xylitol, sorbitol, mannitol or the like can be specifically mentioned.

The base used in the solid preparation for injection of the present invention is not particularly restricted, providing it can be a dissolution auxiliary agent in dissolving a block copolymer in water, and such a base may have a proton receiving ability and shows alkalinity when dissolved in water. Specifically, sodium hydroxide, potassium hydroxide, alkali metal salt of weak acid (for example, sodium salt or potassium salt of carbonic acid, phosphoric acid, acetic acid, lactic acid, citric acid, or the like), ammonia or amines such as triethanolamine or the like, is preferable, and sodium hydrogen carbonate, disodium hydrogen phosphate, sodium citrate or sodium hydroxide is more preferable.

The compounding proportion of a block copolymer composed of a hydrophilic polymer structure moiety and a hydrophobic polyamino acid structure moiety in the solid preparation for injection of the present invention is 10% to 85%, preferably 30% to 60% based on the total amount of the preparation.

The amount of an anthracycline anticancer agent in the solid preparation for injection of the present invention is 5 to 100 parts by weight, preferably 10 to 50 parts by weight based on 100 parts by weight of a block copolymer, and its compounding proportion in the preparation is 1% to 50%, preferably 5% to 20% based on the total amount of the preparation.

The amount of a saccharide in the solid preparation for injection of the present invention is 10 to 500 parts by weight, preferably 20 to 200 parts by weight based on 100 parts by weight of a block copolymer, and its compounding proportion in the preparation is 5% to 80%, preferably 25% to 65% based on the total amount of the preparation.

The amount of a base in the preparation of the present invention is 0.1 to 10 parts by weight, preferably 1 to 4 parts by weight based on 100 parts by weight of a block copolymer, and its compounding proportion in the preparation is 0.05% to 5%, preferably 0.5% to 2% based on the total amount of the preparation.

Next, a method of producing a solid preparation for injection of the present invention will be described.

First, a block copolymer composed of a hydrophilic polymer structure moiety and a hydrophobic polyamino acid structure moiety, and a base are added to water, and they are dissolved, if necessary, by heating. The concentration of a block copolymer is 0.01% to 50%, preferably 0.1% to 25%. The amount of a base is an amount by which pH is 6 to 13, preferably 7 to 10, and from 0.1 to 10 parts by weight, preferably 1 to 4 parts by weight based on 100 parts by weight of a block copolymer. A base may be dissolved in water previously, or added simultaneously with a block copolymer or after it. Heating is so effected to give temperatures from 50 to 80°C, preferably 60 to 70°C. After dissolution, quick cooling is conducted down to 40°C or lower. The heating time is preferably as short as possible to suppress the decomposition of a block copolymer at minimum level, and particularly, 1 hour or shorter is preferable.

An anthracycline anticancer agent and a saccharide are added and dissolved in this block copolymer solution. Dissolution is conducted at 5 to 50°C, preferably 15 to 40°C. The amount of an anthracycline anticancer agent is 5 to 100 parts by weight, preferably 10 to 50 parts by weight based on 100 parts by weight of a block copolymer. The amount of a saccharide is 10 to 500 parts by weight, preferably 20 to 200 parts by weight. It may be allowed that an anthracycline anticancer agent and/or a saccharide are separately dissolved in water, and the resulting solutions are combined mutually. If necessary, pH is controlled to give an aqueous solution of a block copolymer-anthracycline anticancer agent complex. For mixing and stirring operations, a propeller stirrer, homomixer, disperser or the like can be used. A homogenizer, high pressure homogenizer or the like, and dedicated machines for micelle formation, emulsification and suspension may be used.

pH of an aqueous solution is controlled, if necessary, to 4 to 9, preferably 5 to 8. As a pH controlling agent, preferable is hydrochloric acid, nitric acid, sodium hydroxide, potassium hydroxide, ammonia, amines such as triethanolamine or the like, or citric acid, acetic acid, tartaric acid, carbonic acid, lactic acid, sulfuric acid, phosphoric acid, or alkali metal salts thereof such as sodium salts, potassium salts or the like, or ammonium salts thereof, and more preferably is hydrochloric acid or sodium hydroxide. Thus obtained aqueous solution is also included in the present invention.

For use as a preparation for injection, it is preferable that an aqueous solution of a block copolymer-anthracycline anticancer agent complex is prepared in an aseptic operation, or filtrated for sterilization after preparation. For leveling the particle size of the micelle of a block copolymer-anthracycline anticancer agent complex, it may be filtrated once or more times before filtration for sterilization. The filtration operation may be conducted under positive pressure. This operation may be conducted in one filtrating operation using a filter installed together with a filter for filtration under sterilization. For filtration for leveling the particle size of a micelle, membrane filters such as usual cellulose-based or synthetic polymer-based membranes and the like can be used, and the minimum pore size is preferably 0.05 to 0.5 µm.

The filtrated aqueous solution is dried to give a solid powder by usual methods. As the drying method, for example, a lyophilization method, spray drying method and the like are mentioned, and a lyophilization is preferable. A lyophilizer usually used for producing medicinal preparations can be used, and the aqueous solution is filled in a vial or ample tube and lyophilized, and sealed to give a solid preparation for injection.

Solid preparations for injection produced by the above-mentioned production method are also included in the present invention.

The solid preparation for injection of the present invention which is a lyophilized preparation is also included in the present invention.

Further, the present invention also includes an aqueous solution preparation of a block copolymer micelle composed of a hydrophobic polyamino acid structure moiety and a hydrophilic polymer structure moiety containing an anthracycline anticancer agent in a hydrophobic inner core, obtained by dissolving the above-mentioned solid preparation for injection in infusion.

As the infusion in the present invention, usually used infusion such as an injection solvent, physiological saline, glucose liquid and the like are mentioned. Its pH is from 4 to 9, preferably 5 to 8. The aqueous solution preparation of the present invention may contain pharmaceutically acceptable pH controlling agent and isotonizing agent used for allowing the dissolved liquid to have desired pH and osmotic pressure.

As the above-mentioned pH controlling agent, hydrochloric acid, sodium hydroxide, potassium hydroxide, ammonia, amines such as triethanolamine or the like, and citric acid, acetic acid, tartaric acid, carbonic acid, lactic acid, sulfuric acid, phosphoric acid, or alkali metal salts thereof such as sodium salt, potassium salt or the like, or ammonium salts thereof, or a combination containing an acid and its salt having a buffering action is preferable, i.e. a combination containing citric acid, phosphoric acid or their salts is more preferable. By use of the above-mentioned pH controlling agent, pH of an aqueous solution is controlled to 4 to 9, preferably 5 to 8. As the above-mentioned isotonizing agent, sodium chloride, glycerin, monosaccharide such as glucose, fructose, mannitol, xylitol and the like, disaccharide such as maltose and the like are mentioned, and preferable are sodium chloride, glycerin and mannitol. An isotonizing agent is used in a concentration such that the osmotic pressure ratio of dissolved liquid to physiological saline is 0 to 3, preferably about 0.5 to 2. Its concentration is preferably 0.5% to 1.8% for sodium chloride, 1.3% to 5% for glycerin, and 2.5% to 10% for mannitol.

Further, a block copolymer micelle aqueous solution of pH 4 to 9 obtained by dissolving a block copolymer composed of a hydrophilic polymer structure moiety and a hydrophobic polyamino acid structure moiety bonded by an anthracycline anticancer agent, an anthracycline anticancer agent, a saccharide and a base in water or an aqueous solution containing a salt is also included in the present invention. In this aqueous solution, the block copolymer forms a micelle containing a hydrophilic polymer structure moiety as an outer side moiety and a hydrophobic polyamino acid structure moiety bonded by an anthracycline anticancer agent as an inner side moiety, and an anthracycline anticancer agent is mainly contained in its hydrophobic inner core. As the block copolymer, anthracycline anticancer agent, a saccharide and a base, those which can be used in the above-mentioned solid preparation for injection are mentioned, and also as the amount and amount ratio thereof, the above-mentioned ranges are mentioned.

### EXAMPLES

The effect of the invention will be specifically illustrated by examples and test examples below, however, the scope of the invention is not limited to them.

### Example 1

1000 mg of a block copolymer obtained according to a reference example described later, and 18.5 mg of sodium hydrogen carbonate were added to 20 mL of an injection solvent, and dissolved with stirring at 60 to 70°C, then, the solution was cooled to room temperature. Separately, 200 mg of doxorubicin hydrochloride and 1000 mg of sucrose were dissolved with stirring in 40 mL of an injection solvent. Both solutions were combined and pH thereof was controlled to 6 with sodium hydroxide and hydrochloric acid, then, the total amount was controlled to 100 mL with an injection solvent. The solution was filtrated through a membrane filter having a pore size of 0.45 µm, then, filtrated for sterilization through a membrane filter having a pore size of 0.2 µm. The solution was filled in vials each in an amount of 5 mL, lyophilized, then, the vials were sealed, to give solid preparations for injection. 5 mL of an injection solvent was added to this preparation to re-dissolve the preparation, obtaining an aqueous solution of a block copolymer-doxorubicin complex. The average micelle diameter of this complex was 27 nm by dynamic light scattering method.

### Example 2

1000 mg of a block copolymer (the same as in Example 1) and 8.8 mg of sodium hydroxide were added to 20 mL of an injection solvent, and dissolved with stirring at 60 to 70°C, then, the solution was cooled to room temperature. Separately, 200 mg of doxorubicin hydrochloride and 800 mg of trehalose were dissolved with stirring in 40 mL of an injection solvent. Both solutions were combined and pH thereof was controlled to 6 with sodium hydroxide and hydrochloric acid, then, the total amount was controlled to 100 mL with an injection solvent. The solution was filtrated through a membrane filter having a pore size of 0.45 µm, then, filtrated for sterilization through a membrane filter having a pore size of 0.2 µm. The solution was filled in vials each in an amount of 5 mL, lyophilized, then, the vials were sealed, to give solid preparations for injection. 5 mL of an injection solvent was added to this preparation to re-dissolve the preparation, obtaining an aqueous solution of a block copolymer-doxorubicin complex. The average micelle diameter of this complex was 28 nm by dynamic light scattering method.

### Example 3

1000 mg of a block copolymer (the same as in Example 1) and 18.5 mg of sodium hydrogen carbonate were added to 20 mL of an injection solvent, and dissolved with stirring at 60 to 70°C, then, the solution was cooled to room temperature. Separately, 200 mg of doxorubicin hydrochloride and 1000 mg of maltose were dissolved with stirring in 40 mL of an injection solution. Both solutions were combined and pH thereof was controlled to 6 with sodium hydroxide and hydrochloric acid, then, the total amount was controlled to 100 mL with an injection solvent. The solution was filtrated through a membrane filter having a pore size of 0.45 µm, then, filtrated for sterilization through a membrane filter having a pore size of 0.2 µm. The solution was filled in vials each in an amount of 5 mL, lyophilized, then, the vials were sealed, to give solid preparations for injection. 5 mL of an injection solvent was added to this preparation to re-dissolve the preparation, obtaining an aqueous solution of a block copolymer-doxorubicin complex. The average micelle diameter of this complex was 29 nm by dynamic light scattering method.

### Example 4

1000 mg of a block copolymer (the same as in Example 1) and 9.6 mg of sodium hydroxide were added to 20 mL of an injection solvent, and dissolved with stirring at 60 to 70°C, then, the solution was cooled to room temperature. Separately, 200 mg of doxorubicin hydrochloride and 800 mg of lactose were dissolved with stirring in 40 mL of an injection solvent. Both solutions were combined and pH thereof was controlled to 6 with sodium hydroxide and hydrochloric acid, then, the total amount was controlled to 100 mL with an injection solvent. The solution was filtrated through a membrane filter having a pore size of 0.45 µm, then, filtrated for sterilization through a membrane filter having a pore size of 0.2 µm. The solution was filled in vials each in an amount of 5 mL, lyophilized, then, the vials were sealed, to give solid preparations for injection. 5 mL of an injection solvent was added to this preparation to re-dissolve the preparation, obtaining an aqueous solution of a block copolymer-doxorubicin complex. The average micelle diameter of this complex was 25 nm by dynamic light scattering method.

### Example 5

1000 mg of a block copolymer (the same as in Example 1) of the formula (1) and 20 mg of sodium hydrogen carbonate were added to 20 mL of an injection solvent, and dissolved with stirring at 60 to 70°C, then, the solution was cooled to room temperature. Separately, 200 mg of doxorubicin hydrochloride and 600 mg of sucrose were dissolved with stirring in 40 mL of an injection solvent. Both solutions were combined and pH thereof was controlled to 5.5 with sodium hydroxide and hydrochloric acid, then, the total amount was controlled to 100 mL with an injection solvent. The solution was filtrated through a membrane filter having a pore size of 0.45 µm, then, filtrated for sterilization through a membrane filter having a pore size of 0.1 µm. The solution was filled in vials each in an amount of 5 mL, lyophilized, then, the vials were sealed, to give solid preparations for injection. 5 mL of an injection solvent was added to this preparation to re-dissolve the preparation, obtaining an aqueous solution of a block copolymer-doxorubicin complex. The average micelle diameter of this complex was 36 nm by dynamic light scattering method.

### Example 6

1000 mg of a block copolymer (the same as in Example 1) of the formula (1) and 20 mg of sodium hydrogen carbonate were added to 20 mL of an injection solvent, and dissolved with stirring at 60 to 70°C, then, the solution was cooled to room temperature. Separately, 200 mg of doxorubicin hydrochloride and 1500 mg of sucrose were dissolved with stirring in 40 mL of an injection solvent. Both solutions were combined and pH thereof was controlled to 5.5 with sodium hydroxide and hydrochloric acid, then, the total amount was controlled to 100 mL with an injection solvent. The solution was filtrated through a membrane filter having a pore size of 0.45 µm, then, filtrated for sterilization through a membrane filter having a pore size of 0.1 µm. The solution was filled in vials each in an amount of 5 mL, lyophilized, then, the vials were sealed, to give solid preparations for injection. 5 mL of an injection solvent was added to this preparation to re-dissolve the preparation, obtaining an aqueous solution of a block copolymer-doxorubicin complex. The average micelle diameter of this complex was 35 nm by dynamic light scattering method.

### Comparative Example 1

1000 mg of a block copolymer (the same as in Example 1) and 20 mg of sodium hydrogen carbonate were added to 20 mL of an injection solvent, and dissolved with stirring at 60 to 70°C, then, the solution was cooled to room temperature. Separately, 200 mg of doxorubicin hydrochloride was dissolved with stirring in 40 mL of an injection solvent. Both solutions were combined and pH thereof was controlled to 6 with sodium hydroxide and hydrochloric acid, then, the total amount was controlled to 100 mL with an injection solvent. The solution was filtrated through a membrane filter having a pore size of 0.45 µm, then, filtrated for sterilization through a membrane filter having a pore size of 0.2 µm. The solution was filled in vials each in an amount of 5 mL, lyophilized, then, the vials were sealed, to give solid preparations for injection. 5 mL of an injection solvent was added to this preparation to re-dissolve the preparation, obtaining an aqueous solution of a block copolymer-doxorubicin complex. The average micelle diameter of this complex was 165 nm by dynamic light scattering method.

### Test Example 1 (re-dissolving property)

The solid preparations for injection of the present invention in Examples 1 to 4 and the solid preparation for injection in Comparative Example 1 were stored at 40°C for 1 week, and 5 mL of an injection solvent was added to re-dissolve the preparations. The re-dissolving time in this operation was compared. The test was conducted according to the method by Sugihara et al. (M. Sugihara, "Iyakuhin housou no shiyousei to sono hyouka", p 138, Kodansha Ltd. Publishers, 1996). Also the average micelle diameter after re-dissolving was measured by dynamic light scattering method. The results are shown in Table 1. The solid preparations for injection of the present invention in Examples 1 to 4 were stable showing no change by heat in dissolvability and in average micelle diameter. The preparation in Comparative Example 1 containing no saccharide became un-redissolvable by heat.

Test method
(1) 5 mL of an injection solvent of 20°C was added to the preparations in vials of the examples and to the preparations in vials of the comparative example.
(2) The mixtures were shaken for 15 seconds by hands at an amplitude of 40 cm and a speed of 2 reciprocations/second, and allowed to stand still for 5 seconds, and dissolution conditions were observed.
(3) Shaking for 15 seconds and left for 5 seconds were repeated, and the total shaking time was used as redissolving time.

**Table 1**

| Preparation | Saccharide | Redissolving test in production time (micelle diameter) | Redissolving test in after storage time (micelle diameter) |
|---|---|---|---|
| Example 1 | sucrose | 15 sec (27 nm) | 15 sec (29 nm) |
| Example 2 | trehalose | 15 sec (28 nm) | 15 sec (33 nm) |
| Example 3 | maltose | 15 sec (29 nm) | 30 sec (34 nm) |
| Example 4 | lactose | 15 sec (25 nm) | 30 sec (40 nm) |
| Comparative Example 1 | no addition | 30 sec (165 nm) | un-dissolvable (un-measurable) |
| Un-dissolvable: no dissolving within 60 seconds | | | |

### Test Example 2 (stability)

The solid preparation for injection of the present invention in Example 1 was stored at 25°C for 6 months, and its stability was investigated by appearance, remaining ratio, re-dissolving time and average micelle diameter after storage. The results are shown in Table 2. The solid preparation for injection of the present invention was stable even after storage for a long period of time, and judged to be clinically applicable.

**Table 2**

| Preparation | Appearance | Remaining ratio | Redissolving time | Average micelle diameter |
|---|---|---|---|---|
| Example 1 | No change | 97.8% | 15 sec | 27 nm |

### Reference Example

23.32 g of one-end methoxy-one-end 3-aminopropoxyl polyethylene glycol (CH₃(OCH₂CH₂)ₙO(CH₂)₃NH₂ (molecular weight: about 5000) was dissolved in 464 mL of dimethylsulfoxide (DMSO) , and the mixture was heated up to 35°C. 42.87 g of β-benzyl-L-aspartate-N-carboxylic anhydride (BLA-NCA) was added to this, and they were reacted for 22 hours. The reaction mixture was dropped into a mixed solvent of 3.73 liter of diisopropyl ether (IPE) and 0.93 liter of ethanol (EtOH), and the deposited precipitate was filtrated, and washed with a mixed solution of IPE and EtOH (4:1) and IPE, then, dried in vacuo, to obtain 54.29 g of a one-end methoxypolyethylene glycol-poly(β-benzyl L-aspartate) copolymer (number of aspartic acid unit: 29.0).

52.85 g of the resulted one-end methoxypolyethylene glycol-poly (β-benzyl L-aspartate)copolymer was dissolved in 529 mL of dimethylformamide, and the solution was heated at 35°C. To this was added 2.50 mL of acetic anhydride, and they were reacted for 3 hours. The reaction mixture was dropped into a mixed solvent of 4.76 liter of diisopropyl ether (IPE) and 0.53 liter of ethanol (EtOH), and the deposited precipitate was filtrated, and washed with a mixed solution of IPE and EtOH (9:1) and IPE, then, dried in vacuo, to obtain 51.67 g of a one-end Methoxypolyethylene glycol-poly(β-benzyl L-aspartate) copolymer N-acetylated compound.

50.19 g of the resulted one-end methoxypolyethylene glycol-poly(β-benzyl L-aspartate)copolymer N-acetylated compound was reacted with 753 mL of acetonitrile and 2.16 liter of 0.2 N sodium hydroxide solution for 5 hours. The reaction mixture was neutralized with 2N hydrochloric acid, then, concentrated under reduced pressure to remove acetonitrile, then, extracted with 1.2 liter of ethyl acetate three times. The aqueous layer was concentrated, then, purified by a HP-20 SS column (manufactured by Mitsubishi Chemical Co. Ltd.) and a Dowex 50W8 column (manufactured by Dow Chemical Co. Ltd.), further, concentrated under reduced pressure, then, lyophilized. The resulted lyophilized product was dissolved in 320 mL of dimethyl-formamide (DMF), and the solution was dropped into a mixed solvent of 2.56 liter of hexane and 0.64 liter of ethyl acetate. The deposited precipitate was filtrated, and washed with a mixed solution of hexane and ethyl acetate (4:1), and hexane, then, dried in vacuo, to obtain 33.20 g of a one-end Methoxypolyethylene glycol-polyaspartic acid copolymer N-acetylated compound.

28.85 g of the resulted one-end methoxypolyethylene glycol-polyaspartic acid copolymer N-acetylated compound was dissolved in 577 mL of dimethylformamide, and the solution was heated at 35°C. To this was added 19.75 g of dicyclohexylcarbodiimide (DCC) and 11.01 g of N-hydroxysuccinimide (HOSu), and they were reacted for 1 hour. The resulted dicyclohexylurea was filtrated through a cotton plug. The resulted filtrate was diluted with 2.3 liter of ethyl acetate, then, 3.5 liter of hexane was added to this. The deposited precipitate was filtrated, and washed with a mixed solution of hexane and ethyl acetate (3:1), then, dried in vacuo, to obtain 33.82 g of a one-end Methoxy-polyethylene glycol-polyaspartic acid copolymer N-acetylated compound activated by esterification with HOSu.

33.73 g of the resulted one-end methoxypolyethylene glycol-polyaspartic acid copolymer N-acetylated compound activated by esterification with HOSu was dissolved in 1.35 liter of dimethylformamide, and the mixture was heated at 35°C. 26.13 g of doxorubicin hydrochloride was added as it was in the form of powder, and suspended in the reaction liquid, then, 8.16 mL of triethylamine was added, and they were reacted for 1 hour. The reaction mixture was dropped into a mixed solvent of 4.0 liter of ethyl acetate and 16.0 liter of hexane, and the deposited precipitate was filtrated, and washed with a mixed solution of hexane and ethyl acetate (3:1), then, dried in vacuo. After that, the resulted precipitate was suspended in 590 mL of acetonitrile, then, 1780 mL of water was added, and the mixture was stirred with heating at 35°C. After confirmation of dissolving of the precipitate, the solution was stirred for 1 hour, then, the reaction solution was concentrated under reduced pressure to remove acetonitrile, and lyophilized. The resulted lyophilized product was further purified, to obtain 45.39 g of a one-end methoxypolyethylene glycol-polyaspartic acid copolymer N-acetylated compound-doxorubicin condensate. The proportion of doxorubicin bonding part in this block copolymer was about 47%.

### EFFECTS OF THE INVENTION

The present invention has provided, for the first time, a solid preparation for injection of a micelle medical preparation containing doxorubicin which is a clinically useful anticancer agent, made of a block copolymer composed of a hydrophilic polymer structure moiety and a hydrophobic polyamino acid structure moiety, which is stable for a long period of time, manifests excellent redissolvability, providing safety of additives, and clinically applicable, and a method of producing the same, requiring no special apparatus for an organic solvent, irradiation with ultrasonic wave and dialysis, capable of effecting production by a combination of usual production processes, and capable of being conducted industrially.

The above-mentioned solid preparation for injection is a solid composition obtained from a block copolymer-anthracycline anticancer agent complex composed of a block copolymer and an anthracycline anticancer agent, and in use, dissolved in infusion to give an aqueous solution of a micelle of the block copolymer-anthracycline anticancer agent complex.

## Claims

1. A solid preparation for injection comprising a block copolymer composed a hydrophilic polymer structure moiety and a hydrophobic polyamino acid structure moiety, an anthracycline anticancer agent, a saccharide and a base.

2. The solid preparation for injection according to Claim 1, wherein the anthracycline anticancer agent is doxorubicin or its salt and the saccharide is disaccharide.

3. The solid preparation for injection according to Claim 1 or 2, wherein the saccharide includes one or more compounds selected from the group consisting of sucrose, trehalose, maltose and lactose.

4. The solid preparation for injection according to any one of Claims 1 to 3, wherein the base includes one or more compounds selected from the group consisting of sodium hydrogen carbonate, disodium hydrogen phosphate, sodium citrate and sodium hydroxide.

5. The solid preparation for injection according to any one of Claims 1 to 4, wherein the hydrophilic polymer structure moiety in the block copolymer is a polyethylene oxide derivative, and the hydrophobic polyamino acid structure moiety is polyaspartic acid having a side chain to which an anthracycline anticancer agent is bonded.

6. The solid preparation for injection according to Claim 5, wherein the anthracycline anticancer agent bonded to a side chain of the hydrophobic polyamino acid structure moiety is doxorubicin.

7. The solid preparation for injection according to Claim 1, wherein the content of the anthracycline anticancer agent is 5 to 100 parts by weight, the content of the saccharide is 10 to 500 parts by weight and the content of the base is 0.1 to 10 parts by weight, based on 100 parts by weight of the block copolymer composed a hydrophilic polymer structure moiety and a hydrophobic polyamino acid structure moiety.

8. The solid preparation for injection according to Claim 1, wherein the proportions of the components are as described below:
| | |
|---|---|
| block copolymer composed a hydrophilic polymer structure moiety and a hydrophobic polyamino acid structure moiety | 10 to 85 parts by weight |
| anthracycline anticancer agent | 1 to 50 parts by weight |
| saccharide | 5 to 80 parts by weight |
| base | 0.05 to 5 parts by weight. |

9. The solid preparation for injection according to Claim 8, wherein the anthracycline anticancer agent is doxorubicin or salt thereof, the saccharide is sucrose, trehalose, maltose or lactose, and the base is sodium hydrogen carbonate, disodium hydrogen phosphate, sodium citrate or sodium hydroxide.

10. The solid preparation for injection according to any one of Claims 1 to 9, wherein the solid preparation for injection is a lyophilized preparation.

11. A block copolymer micelle preparation composed of a hydrophilic polymer structure moiety and a hydrophobic polyamino acid structure moiety containing an anthracycline anticancer agent in a hydrophobic inner core, obtained by dissolving the solid preparation for injection according to any one of Claims 1 to 10 in infusion.

12. A block copolymer micelle solution of pH 4 to 9, comprising a block copolymer composed of a hydrophilic polymer structure moiety and a hydrophobic polyamino acid structure moiety bonded by an anthracycline anticancer agent, an anthracycline anticancer agent, a saccharide and a base.

13. A process for producing a solid preparation for injection, comprising dissolving a block copolymer composed of a hydrophilic polymer structure moiety and a hydrophobic polyamino acid structure moiety, an anthracycline anticancer agent, a saccharide and a base in water, and then drying them to give solid.

14. The process for producing a solid preparation for injection according to Claim 13, wherein when dissolving a block copolymer composed of a hydrophilic polymer structure moiety and a hydrophobic polyamino acid structure moiety in water, a base is added and the mixture is heated.

15. The process for producing a solid preparation for injection according to Claim 14, wherein the heating temperature is 50 to 80°C.

16. The process for producing a solid preparation for injection according to any one of Claims 13 to 15, comprising dissolving a block copolymer composed of a hydrophilic polymer structure moiety and a hydrophobic polyamino acid structure moiety, an anthracycline anticancer agent, a saccharide and a base in water, and then filtrating through a membrane filter.

17. The process for producing a solid preparation for injection according to Claim 16, wherein the membrane filter is a cellulose-based or synthetic polymer-based filter and its pore size is 0.05 to 0.5 µm.

18. The micelle solution according to Claim 12, wherein the anthracycline anticancer agent is doxorubicin or its salt and the saccharide is disaccharide.

19. The micelle solution according to Claim 18, wherein the disaccharide includes one or more compounds selected from the group consisting of sucrose, trehalose, maltose and lactose.

20. The micelle solution according to Claim 12, wherein the base includes one or more compounds selected from the group consisting of sodium hydrogen carbonate, disodium hydrogen phosphate, sodium citrate and sodium hydroxide.

21. The micelle solution according to Claim 12, wherein the anthracycline anticancer agent is doxorubicin or its salt, the saccharide is sucrose, trehalose, maltose or lactose, and the base is sodium hydrogen carbonate, disodium hydrogen phosphate, sodium citrate or sodium hydroxide.

22. The micelle solution according to Claim 12, wherein the block copolymer is composed of a hydrophilic polymer structure moiety and a hydrophobic polyamino acid structure moiety bonded by an anthracycline anticancer agent.

23. The micelle solution according to Claim 22, wherein the hydrophilic polymer structure moiety of the block copolymer is a polyethylene oxide derivative and the hydrophobic polyamino acid structure moiety is polyaspartic acid having a side chain to which an anthracycline anticancer agent is bonded.

24. The micelle solution according to Claim 22 or 23, wherein the anthracycline anticancer agent bonded to a side chain of the hydrophobic polyamino acid structure moiety is doxorubicin.

25. A solid preparation for injection obtained by drying the micelle solution according to any one of Claims 18 to 24.
